# EUROPEAN PATENT APPLICATION

(11) **EP 0 803 498 A1**
(43) Date of publication of application: **29.10.1997**
(21) Application number: 97106906.7
(22) Date of filing: 25.04.1997
(51) Int. Cl.: C07C 219/06, C07C 219/08, C07D 295/13, C11D 1/46, C11D 1/62, C11D 1/645

(54) **Polyester, polyquaternary compounds, compositions containing them and their use as fabric softener**

(30) Priority: 26.04.1996 US 638615
(71) Applicant: WITCO CORPORATION, Greenwich, Connecticut 06831-2559 (US)
(72) Inventor: Keys, Robert O., Columbus, Ohio 43235 (US); Friedli, Floyd E., Dublin, Ohio 43017 (US)
(74) Representative: Hansen, Bernd, Dr. Dipl.-Chem.

(57) **Abstract**

Disclosed are compounds of the formula (1) 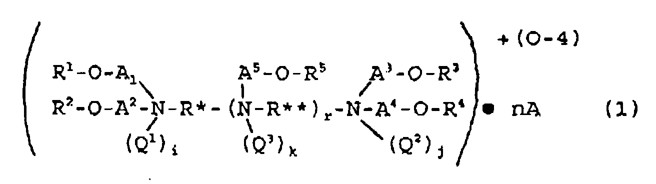 wherein R^{*} and R^{**} are C₂₋₁₂ alkyl, each of A¹⁻⁵ is C₂₋₄ alkyl, each of R¹⁻⁵ is -H or (C₉₋₂₁ alkyl or alkenyl)-C(O)-, each of Q¹⁻³ is -H,-CH₃, -C₂H₅ or -CH₂CH₂COOH; r is 0-2; i, j and each k are each 0-1, and (i+j+k)is 0-4; and n is the number of moles of monovalent anion A to provide zero net charge.

## Description

### BACKGROUND OF THE INVENTION

The present invention relates to amine and quaternary ammonium compounds useful as, for instance, fabric softeners, paper debonders, hair conditioners, skin conditioners, textile softeners, car wax sprays, and the like.

A variety of quaternary ammonium compounds and diquaternary diammonium compounds have in the past been proposed for use in fabric softeners for home use and/or for industrial and institutional use. In general, such compounds exhibit properties which present some difficulty in the manufacture, formulation use, biodegradability and/or environmental compatibility of these compositions. Many of the conventional compositions used for these functions, even if completely biodegradable with time, do not biodegrade as rapidly as could be desired and are thus not considered readily biodegradable. Several of the commercial readily biodegradable softeners, conditioners and debonders do not function as effectively as the conventionally used, less biodegradable products. Thus, increased amounts of such less effective, more readily biodegradable products (such as softeners) must be employed, to maintain effective levels of performance; this decreases the cost effectiveness of the product.

The color and the odor of the products also pose problems with many biodegradable softener raw materials. Light color and low odor are essential to obtaining customer acceptance and to achieving stable, acceptable long term product aesthetic properties.

The relatively poor solubility also contributes to difficulties in dispersibility of the fabric softener actives into water, and dispersibility of the formulated fabric softener product into the washing machine.

In addition, there is increasing interest in obtaining fabric softener formulations which are translucent or transparent liquids, even to the point of obtaining a crystal clear dispersion when the formulation is dispensed and dispersed into rinse water (even at levels of 50-100 ppm active in water). Clear formulations offer reduced staining of the fabric, improved dispersibility, and greatly improved rewetting of the fabric or other substrate. Discovery of such compositions requires careful identification of proper quaternary and/or polyquaternary ammonium compounds, together with appropriate additives such as solvents and cosolvents which act together to achieve the desired appearance.

Thus, there remains a need for identification of new amine and ammonium derivatives, and particular diquaternary and polyquaternary derivatives, which are useful as fabric softeners and which are biodegradable, highly effective in softening, debonding, conditioning and the like, and yet avoid these problems upon manufacture, formulation and use.

It is also desirable for the active agents used in hair and skin conditioners, paper debonding compositions, textile softeners, and the like, to be readily biodegradable and to exhibit a satisfactorily high activity. Conventional products have to date not been able to exhibit both properties to a high degree, thus necessitating acceptance of reduced biodegradability or reduced activity. There is thus still a need for compounds exhibiting levels of activity as conditioners, paper debonders, and so on, as the case may be, which are comparable or superior to conventionally employed actives, while also exhibiting ready biodegradability.

### SUMMARY OF THE INVENTION

The present invention achieves these objectives and also exhibits the properties and advantages described herein.

One aspect of the present invention is compounds of the formula (1) 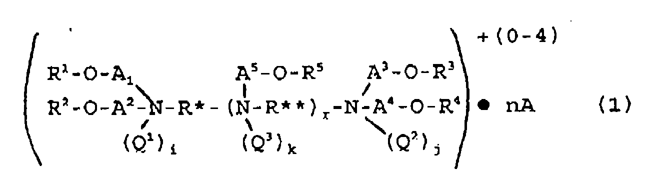 wherein each of R* and R** is independently a linear, branched or cyclic alkyl group containing 2 to 12 carbon atoms, wherein no two nitrogen atoms are separated by fewer than 2 carbon atoms;
each of A¹, A², A³, A⁴ and each occurrence of A⁵ is independently straight or branched alkyl containing 2 to 4 carbon atoms;
each of R¹, R², R³, and R⁴ and each occurrence of R⁵ is independently -H or R^{A}C(=O)- wherein R^{A} is straight or branched alkyl or alkenyl containing 9 to 21 carbon atoms and 0 to 4 carbon-carbon double bonds; provided that at least one of R¹, R², R³, R⁴ or R⁵ is R^{A}C(=O)-;
each of Q¹, Q² and Q³ is -H,-CH₃, -C₂H₅, or - CH₂CH₂COOH;
r is 0-2;
i is 0-1, j is 0-1, each k is 0-1 and the sum of (i+j+k) is 0-4; or a 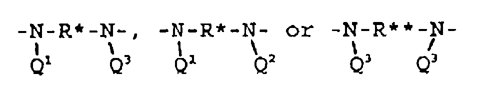 moiety is 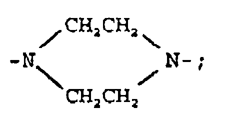
A is a monovalent anion; and n is the number of moles of A needed to give the compound of formula (1) zero net charge.

One embodiment of formula (1) is compounds of the formula (Q) 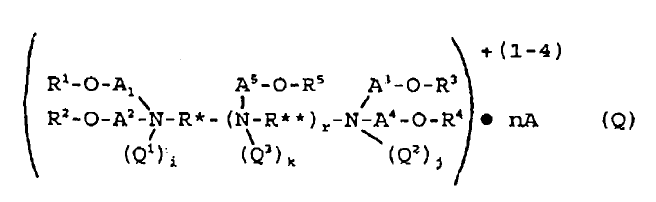 wherein each of R* and R** is independently a linear, branched or cyclic alkyl group containing 2 to 12 carbon atoms, wherein no two nitrogen atoms are separated by fewer than 2 carbon atoms;
each of A¹, A², A³, A⁴ and each occurrence of A⁵ is independently straight or branched alkyl containing 2 to 4 carbon atoms;
each of R¹, R², R³, and R⁴ and each occurrence of R⁵ is independently -H or R^{A}C(=O)- wherein R^{A} is straight or branched alkyl or alkenyl containing 9 to 21 carbon atoms and 0 to 4 carbon-carbon double bonds; provided that at least one of R¹, R², R³, R⁴ or R⁵ is R^{A}C(=O)-;
each of Q¹, Q² and Q³ is -CH₃, -C₂H₅, or - CH₂CH₂COOH;
r is 0-2;
i is 0-1, j is 0-1, each k is 0-1 and the sum of (i+j+k) is 1-4; or a 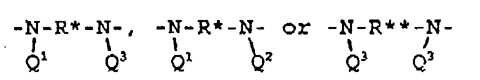 moiety is 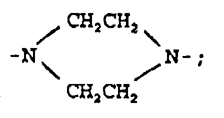
A is a monovalent anion; and n is the number of moles of A needed to give the compound of formula (Q) zero net charge.

Another embodiment of formula (1) is compounds wherein substituents Q¹, Q² and Q³ are not present, or there is one or more substituents selected from Q¹, Q² and Q³ and any such substituent present is -H.

Another aspect of the present invention comprises mixtures of two or more compounds of the foregoing formulas (1) and/or (Q).

Another aspect of the present invention comprises liquid compositions, useful for instance as fabric softeners, comprising (a) a component selected from the group consisting of compounds of formula (1) and mixtures thereof, which may be a cationic component selected from the group consisting of compounds of formula (Q) and mixtures thereof; (b) water; and (c) optionally, one or more solvents or co-solvents.

### DETAILED DESCRIPTION OF THE INVENTION

The present invention is directed to novel compounds, and particularly to advantageous liquid formulations containing such compounds.

### PREFERRED COMPOUNDS AND COMPOSITIONS

As will be appreciated, a particularly preferred embodiment of the present invention comprises mixtures of compounds corresponding to formula (1), such that degrees of quaternization, or protonation, (including unquaternized, unprotonated compounds), degrees of esterification, and chain lengths and molecular weights, of different molecules within the mixture will differ such that the respective properties can be represented as an average over all the molecules present in the mixture. In such mixtures, then, properties such as the degree of quaternization can be expressed as average values which may lie between integer whole numbers.

Referring to formula (1), the groups depicted as Q¹, Q² and Q³ represent substituents which, when present, protonate or quaternize the respective nitrogen atoms to which they are bonded. While the structure of formula (1) embraces compounds ranging from compounds wherein no nitrogen atom is quaternized or protonated with a corresponding Q substituent, through compounds wherein every nitrogen atom is quaternized or protonated with a Q substituent, one preferred embodiment is compounds wherein the compound of formula (1) should be partially quaternized. By 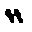 partially*"* is meant that at least one nitrogen atom on the molecule as depicted in formula (1) does not have a Q substituent attached to it. In particular, in mixtures of compounds which are diamines (i.e., in formula (1), the subscript r is zero), there is on average in the mixture preferably 1-2, and more preferably 1.5-2, quaternizing groups Q per molecule.

Another preferred embodiment is compounds, and mixtures thereof, in which there are no Q substituents, or only Q substituents are present which are -H. Such Q substituents can be present in a degree such that all nitrogen atoms are protonated, or fewer than all nitrogen atoms are protonated ( 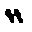 full*"* and 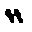 partial*"* protonation, respectively).

The compounds of formula (1) may also have any of a range of degrees of esterification. As shown in formula (1), the degree of esterification can range from 1 to 4 for diamines, and up to 5 or 6 for triamines or tetramines, respectively. The preferred degree of esterification, that is, the total number of R^{A} groups present, preferably ranges from 2 to 4. This is true especially for diamines, wherein a preferred degree of esterification is from 1 to 3 for clear, translucent formulations and 3 to 4.5 for easily dispersible formulations, and the more preferred degree of esterification is about 2.5. Any desired degree of esterification can be attained by reaction between the polyhydroxy-substituted precursor and a stoichiometrically appropriate amount of the corresponding carboxylic acid(s). For instance, even a noninteger target degree of esterification such as 2.5 can be attained by reaction to form a mixture containing equal amounts of triester and diester.

The respective R^{A}C(O)- acyl groups can all have the same chain lengths. More preferably they have several chain lengths and degrees of carbon-carbon unsaturation, reflecting the fact that the fatty acyl groups can be derived from naturally occurring sources which contain mixtures of fatty acids with differing chain lengths and differing degrees of carbon-carbon unsaturation. Examples of much sources include tallow fatty acids, coco fatty acids, and canola fatty acids (including fatty acids derived from partially hydrogenated canola oil).

Referring to formula (1), the molecules shown can be diamines, triamines, or tetramines. For many purposes, the diamines are preferred. The R* bridge linking the two nitrogen atoms of the diamine can contain 2 to 12 carbon atoms, and preferably contains more than 2 carbon atoms. One particularly preferred R* group is alkyl containing 6 to 12 carbon atoms, such as linear hexamethylene. Other useful groups can contain up to 10 carbon atoms between the nitrogen atoms, or anywhere from 3 to 10 carbon atoms presenting straight or branched alkyl structures.

The various moieties depicted at formula (1) as A¹⁻⁵ can each, independently, be ethyl, propyl (straight or branched) or butyl (straight or branched). Preferably, each of the A¹⁻⁵ groups present is the same, for ease of synthesis. It is also preferred that each of A¹⁻⁵ is ethyl or propyl (especially isopropyl).

Further preferred embodiments of compounds of formula (1) are described herein with respect to the various formulation capabilities of the products of this invention.

The compounds of formula (1), when quaternized and/or protonated to any degree, include a monovalent anion A which is present in a number of moles equal to the total positive charge of the nitrogen-containing cation. The anion A can be any anion which is not deleterious to the properties of the overall compound. Preferred examples include halide anions, particularly chloride; as well as methyl sulfate and ethyl sulfate.

### SYNTHESIS

Compounds of formula (1) can readily be synthesized from readily available starting materials using reaction procedures and conditions quite familiar to those of ordinary skill in this art. The preferred procedure begins with a diamine, triamine or tetramine of the formula H₂N-R*-(NH-R**)ᵣ-NH₂. This polyamine compound is next alkoxylated. The alkoxylating reactants are ethylene oxide, propylene oxide, or butylene oxide, or (less preferred) a mixture thereof, depending on the desired choice of alkyl groups to become attached to the respective nitrogen atoms. The number of moles of alkylene oxide reactant, and the reaction conditions, are established such that the terminal primary amine hydrogen atoms are disubstituted with hydroxy alkyl, and each of the secondary amines, if any, is monosubstituted with hydroxy alkyl.

Next, the alkoxylated product is esterified by reaction of fatty acids with the respective hydroxyl groups resulting from the alkoxylation. As indicated above, the molecule may be completely esterified, but it is preferred for formulation reasons to achieve only partial esterification. There should however be at least one ester group per molecule of formula (1).

The esterification is carried out with carboxylic acids of the formula R^{A}C(O)OH, wherein R^{A} is straight or branched alkyl or alkenyl containing 9 to 21 carbon atoms and 0 to 4 carbon-carbon double bonds. While the esterification can be carried out with an appropriate quantity of one such carboxylic acid, it is preferred for reasons of economy, product performance and convenience to employ mixtures of carboxylic acids each corresponding to the formula R^{A}C(O)OH. For instance, mixtures of such fatty acids from various animal and vegetable origins are conveniently commercially available. One example of such material is tallow fatty acids, which as is generally known in this field is a mixture of fatty acids predominantly composed of fatty acids containing 14, 16 and 18 carbon atoms, and 0, 1 and 2 degrees of unsaturation. Other preferred sources include coconut fatty acids, and canola fatty acids. Esterification is carried out under conventional conditions, well known to the chemist in this field, allowing for the withdrawal of byproduct water. The number of moles of fatty acid is selected to provide the desired average degree of esterification in the mixture of products formed upon esterification.

Next, if desired, the esterified product or mixture of esterified products is quaternized and/or protonated. As recognized hereinabove, the esterified product can be completely quaternized, but it is preferred to carry out at most partial quaternization only, if any. Quaternization is carried out under conditions well known in this field for quaternization of amines, by reaction of the esterified amine with a suitable quaternizing agent. The quaternizing agents have the formulas Q¹A and Q²A, which can of course be the same and preferably are the same. With triamines and tetramines a second (or third) quaternizing agent Q³A can be used; it is likewise preferably the same as Q¹A and Q²A. Preferably, only one particular quaternizing agent is employed, in which case all of the quaternizing substituents Q will be the same. Preferred quaternizing agents include methyl chloride, dimethyl sulfate and diethyl sulfate.

Protonation can be carried out by reacting the esterified product or mixture of products with an acid of the formula HA, such as hydrochloric acid.

Each of the foregoing reactions can be carried out in solvent or in solvent-free conditions, in each case employing conditions well established for the respective reactions in this field.

### Formulations/Properties

The products as described herein exhibit a number of desirable properties making them particularly suitable for formulation into commercial products such as fabric softeners.

Most notably, the compounds of formula (1) can readily be formulated into useful compositions such as aqueous compositions, which achieve the desired functionality and which are clear, i.e. transparent. This property can be realized at a variety of concentrations of active ingredient, with or even without special solvents or coupling agents.

Other properties are realized as well. For instance, the products are biodegradability. Surprisingly, the embodiments which have no Q substituents, or wherein the only Q substituents are - H, exhibit particularly high biodegradable.

Also, the compounds of formula (1) exhibit advantageous stability, solubility, and freedom from excessively objectionable color and odor.

The color stability is exhibited in formulations wherein the compound or compounds of formula (1) are the only nitrogenous compounds present. Color stability is also exhibited in formulations wherein one or more compounds of formula (1) is present with one or more other quaternary ammonium fabric softening compounds. Thus, the compounds of the present invention improve the color stability of the other quaternary ammonium compounds present.

This property is shown in the following data, in which color stability was assessed by heating samples of each product shown in Table CS below to 110°C in open tubes in an air circulation oven, periodically measuring the color (Gardner), and calculating the color change over time:

**Table-CS**

| Product | Color at: | | | | Rate of color change/day | |
|---|---|---|---|---|---|---|
| | start | 1 day | 2 days | 6 days | At 2 days | At 6 days |
| 1 | 2.5 | 4.5 | 6.0 | 14.5 | 1.75 | 2.0 |
| 2 | 1. | 4.5 | 6.5 | 16. | 2.75 | 2.5 |
| 3 | 1 | 1.5 | 4. | 8.5 | 1.5 | 1.3 |
| 4 | 4. | - | - | 11.5* | - | 1.9* |
| 5 | 2.5 | 5.5 | 6.5 | 12.5 | 2.0 | 1.7 |
| 6 | 3. | 4.5 | 5.5 | 5.5 | 1.25 | 0.4 |
| 7 | 2.5 | 3.5 | 3.5 | 3.5 | 0.5 | 0.17 |
| 8 | 1. | 1. | 2. | 3.5 | 0.5 | 0.4 |
| 9 | 2.5 | 2.5 | 2.5 | 2.5 | 0. | 0. |
| 10 | 1.5 | 1.5 | 1.5 | 1.5 | 0. | 0. |
| 11 | 1.5 | 1.5 | 1.5 | 2.5 | 0 | 0.17 |
| 12 | 2.5 | 5.5 | 7.5 | 11.5 | 2.5 | 1.5 |
| 13 | 1. | - | - | 3.5* | - | 0.62* |
| 14 | 1.5 | - | - | 5.5* | - | 1.0* |

| | | | | | | |
|---|---|---|---|---|---|---|
| * - 4 day readings and averages. | | | | | | |
| Key to product numbers: 1: Soft tallow derived, triethanolamine-ester quaternary. 2: Soft tallow derived, methyl diethanolamine-ester quaternary 3: Canola derived, triethanolamine-ester quaternary 4: Soft tallow derived, diamidoamine quaternary 5: Hexamethylene diamine + 4 moles ethylene oxide + 2.5 moles soft tallow fatty acids + 1 mole dimethylsulfate 6: As 5 except 2 moles dimethyl sulfate. 7: As 6 except 2 moles of canola fatty acid. 8: Bis(hexamethylene)triamine + 5 moles ethylene oxide + 4 moles soft tallow fatty acid + 3 moles dimethylsulfate 9: As 8 except 1.5 moles soft tallow fatty acid 10: As 8 except 2. moles soft tallow fatty acid 11: As 8 except 3. moles canola fatty acid 12: As 8 except 2. moles soft tallow fatty acid and not quaternized 13: 50/50 (wt.%) mixture of 1 and 8 14: 50/50 (wt.%) mixture of 1 and bis(hexamethylene) triamine + 5 moles ethylene oxide + 3.5 moles soft tallow fatty acid + 3 moles dimethylsulfate | | | | | | |

The data show the substantial, significant improvement in color stability which the compounds of the present invention exhibit.
In particular, the compounds of formula (1) exhibit highly satisfactory fabric softening capabilities.

The advantageous features of the compounds of formula (1), as well as mixtures of such compounds, can be particularly realized when they are formulated appropriately into products useable as fabric softeners. It has been found that, regardless of the other components that may be present in the fabric softener formulation, the pH of the formulation as a whole should be below 5, and preferably 2.5 to 4.0, in order to maintain low susceptibility of the ester functionality to hydrolysis in water. The preferred manner for providing the desired pH value lies in addition of small amounts of an acid such as HCl or H₂SO₄ consistent with appropriate adjustment of the average degree of esterification and average degree of quaternization mixture of compounds corresponding to formula (1).

Preferred emulsions useful as fabric softener compositions can contain about 2 to about 80 wt.%, preferably 5 to 30 wt.%, and more preferably 6 to 25 wt.%, of one or more compounds corresponding to formula (1). In general, higher solids contents can be provided more easily with lower degrees of quaternization. On the other hand, higher degrees of quaternization (such as a degree of quaternization approaching 2.0 for a diamine), lead to aqueous formulations wherein the maximum acceptable solids content without excessive solubility problems is lower.

The compounds of formula (1) can be formulated into clear fabric softener compositions with water and one or more of the solvents which are conventionally used in formulating fabric softeners. Examples of such solvents include isopropanol, hexylene glycol and propylene glycol. Such formulations generally comprise 10 wt.% up to 50 wt.% of one or a mixture of compounds of formula (1).

The fabric softening actives of formula (1) can be formulated with greater ease than is encountered with conventional quaternary ammonium fabric softener actives. Most quaternary ammonium compounds exhibit a tendency to form a gel during dilution with water when formulated in clear formulations. However, the compounds of formula (1) show very little gelation, or no gelation at all, during dilution with water when formulated in clear formulations -- even with cold water, which would be expected to provoke gelation. This freedom from a tendency to gel means that compositions including compounds of formula (1) can be prepared at active concentrations of 40 wt.% or higher, i.e. even 50 wt.% or higher. The preparation itself of such formulations is much simpler; and the freedom from gelation means that when a formulator chooses to manufacture a more concentrated product for resale to the consumer, there is no need for special treatment to deal with gel formation. Also, the freedom from gel formation means that the consumer can use the more concentrated product directly into the wash water in the wash or rinse cycle, without concern that a gelled byproduct would form that would reduce softening efficiency and/or leave a deposit on the clothes.

The ability of the compounds of formula (1) to form a clear formulation, at lower and at higher concentrations, extends also to formulations which also contain one or more other quaternary ammonium compounds as fabric softener co-actives.

Additional quaternary ammonium compounds that may be present with the compound or compounds of formula (1) in accordance with the present invention include, but are not limited to, nitrogenous compounds selected from the group consisting of quaternized or acid salt derivatives of:
(i) alkylenediamines including compounds of the formula: 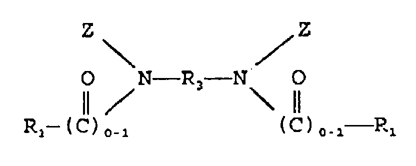 wherein each R₁ is an acyclic alkyl or alkylene C₁₂-C₂₁ hydrocarbon group, each Z is -(R₂O)₀₋₄H, or -R₂H, and R₂ and R₃ are divalent C₁-C₆ alkylene groups;
(ii) substituted imidazoline compounds having the formula: 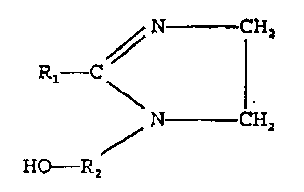
(iii) substituted imidazoline compounds having the formula: 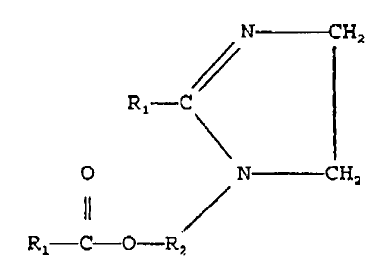 wherein R₁ and R₂ are defined as above;
(iv) reaction products of higher fatty acids with alkylenetriamines in, e.g., a molecular ratio of about 2:1, said reaction products containing compounds of the formula: 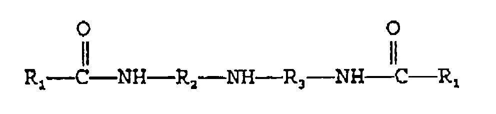 wherein R₁, R₂ and R₃ are defined as above;
(v) substituted imidazoline compounds having the formula: 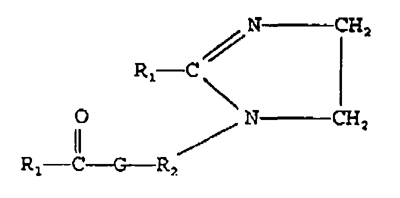 wherein G is -O- or -NH- and R₁ and R₂ are defined as above; and mixtures thereof.
   Preferred examples of compounds of formula (i) are those derived from hydrogenated tallow fatty acids and the hydroxyalkylalkylenediamine N-2-hydroxyethylethylenediamine, such that R₁ is an aliphatic C₁₅-C₂₁ hydrocarbon group, and R₂ and R₃ are divalent ethylene groups.
   A preferred example of compounds of formula (ii) is stearic hydroxyethyl imidazoline wherein R₁ is an aliphatic C₂₁ hydrocarbon group and R₂ is a divalent ethylene group.
   A preferred example of compounds of formula (iv) is N,N''-ditallowalkanoyldiethylenetriamine where R₁ is an aliphatic C₁₅-C₂₁ hydrocarbon group and R₂ and R₃ are divalent ethylene groups.
   A preferred example of compounds of formula (v) is 1-tallowamidoethyl-2-tallowimidazoline wherein R₁ is an aliphatic C₁₅-C₂₁ hydrocarbon group and R₂ is a divalent ethylene group.
   Both N,N''-ditallowalkanoyldiethylenetriamine and 1-tallowethylamido-2-tallowimidazoline are reaction products of tallow fatty acids and diethylenetriamine, and are precursors of the cationic fabric softening agent methyl-1-tallowamidoethyl-2-tallowimidazolinium methylsulfate (see "Cationic Surface Active Agents as Fabric Softeners," R.R. Egan, Journal of the American Oil & Chemicals Society, January 1978, pages 118-121). N,N''-ditallowalkanoyldiethylenetriamine and 1-tallowamidoethyl-2-tallowimidazoline can be obtained from Witco Corporation. Methyl-1-tallowamidoethyl-2-tallowimidazolinium methylsulfate is sold by Witco Corporation under the trade name Varisoft® 475.
   Other useful softening agents include cationic nitrogenous quaternary ammonium compounds and salts, In the cationic nitrogenous salts herein, the anion A^{⊖} provides electrical neutrality. Most often, the anion used to provide electrical neutrality in these salts is a halide, such as chloride, bromide, or iodide. However, other anions can be used, such as methylsulfate, ethylsulfate, acetate, formate, sulfate, carbonate, and the like. Chloride and methylsulfate are preferred herein as the anion A⁻.
   One type of cationic compounds are those containing one long chain acyclic aliphatic C₈-C₂₂ hydrocarbon group, selected from the group consisting of:
(vi) acyclic quaternary ammonium salts having the formula: 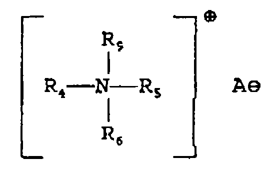 wherein R₄ is an acyclic aliphatic C₈-C₂₂ hydrocarbon group, alkyl, benzyl or (C₄-C₁₈ alkyl)-(OCH₂CH₂)₂₋₃-, R₅ and R₆ are C₁-C₄-saturated alkyl or hydroxyalkyl groups and A^{⊖} is an anion;
(vii) substituted imidazolinium salts having the formula: 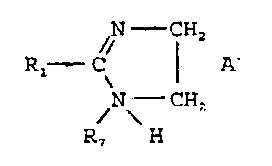 wherein R₁ is an acyclic alkyl or alkylene C₁₂-C₂₁ hydrocarbon group, R₇ is hydrogen or a C₁-C₄ saturated alkyl or hydroxyalkyl group, and A is an anion;
(viii) substituted imidazolinium salts having the formula; 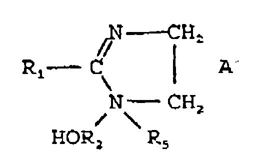 wherein R₁, R₂, R₅ and A are as defined above;
(ix) diquaternaries of the formula (R¹N(Z₂)-(CH₂)₂₋₆-N(Z₃))⁻²·2A⁻
   wherein R¹ and each Z are independently as defined above;
(x) alkylpyridinium salts having the formula: 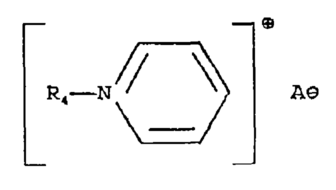 wherein R₄ is an acyclic aliphatic C₈-C₂₂ hydrocarbon group and A^{⊖} is an anion; and
(xi) alkanamide alkylene pyridinium salts having the formula: 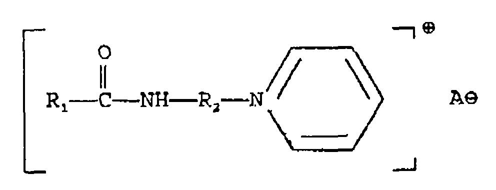 wherein R₁ is an acyclic aliphatic C₁₂-C₂₁ hydrocarbon group, R₂ is a divalent C₁-C₆ alkylene group, and A^{⊖} is an anion; and mixtures thereof.
   Examples of compound (vi) are the monoalkyltrimethylammonium salts such as monotallowtrimethylammonium chloride, mono(hydrogenated tallow)-trimethylammonium chloride, palmityltrimethylammonium chloride and soyatrimethylammonium chloride, sold by Witco Corporation under the trade name Adogen 471, Adogen 441, Adogen 444, and Adogen 415, respectively. In these compounds, R₄ is an acyclic aliphatic C₁₆-C₁₈ hydrocarbon group, and R₅ and R₆ are methyl groups. Mono(hydrogenated tallow)trimethylammonium chloride and monotallowtrimethylammonium chloride are preferred. Other examples of compound (vi) are behenyltrimethylammonium chloride wherein R₄ is a C₂₂ hydrocarbon group and sold under the trade name Kemamine® Q2803-C by Humko Chemical Division of Witco Corporation; soyadimethylethylammonium ethylsulfate wherein R₄ is a C-₁₆-C₁₈ hydrocarbon group, R₅ is a methyl group, R₆ is an ethyl group, and A⁻ is an ethylsulfate anion; and methyl bis(2-hydroxyethyl)octadecylammonium chloride wherein R₄ is a C₁₈ hydrocarbon group, R₅ is a 2-hydroxyethyl group and R₆ is a methyl group.
   An example of compound (viii) is 1-ethyl-1-(2-hydroxyethyl)-2-isoheptadecylimidazolinium ethylsulfate wherein R₁ is a C₁₇ hydrocarbon group, R₂ is an ethylene group, R₅ is an ethyl group, and A⁻ is an ethylsulfate anion.
   Other fabric softening agents useful in the present invention include cationic nitrogenous salts having two or more long chain acyclic aliphatic C₈-C₂₂ hydrocarbon groups or one said group and an arylalkyl group. Examples include:
(xii) acyclic quaternary ammonium salts having the formula: 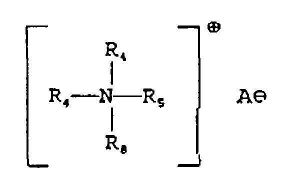 wherein each R₄ is an acyclic aliphatic C₈-C₂₂ hydrocarbon group, R₅ is a C₁-C₄ saturated alkyl or hydroxyalkyl group, R₈ is selected from the group consisting of R₄ and R₅ groups, and A^{⊖} is an anion defined as above;
(xiii) diamido quaternary ammonium salts having the formula: 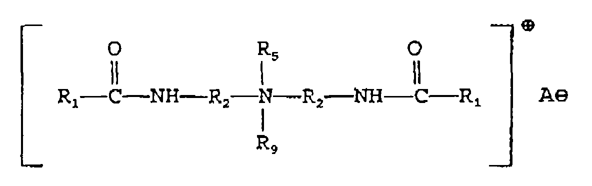 wherein each R₁ is an acyclic alkyl or alkylene C₁₂-C₂₁ hydrocarbon group, each R₂ is a divalent alkylene group having 1 to 3 carbon atoms, R₅ and R₉ are C₁-C₄ saturated alkyl or hydroxyalkyl groups, and A^{⊖} is an anion;
(xiv) alkoxylated diamido quaternary ammonium salts having the formula: 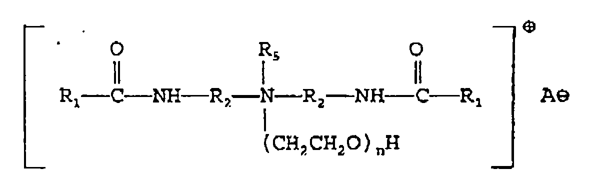 wherein n is equal to 1 to about 5, and R₁, R₂, R₅ and A^{⊖} are as defined above;
(xv) quaternary ammonium compounds having the formula: 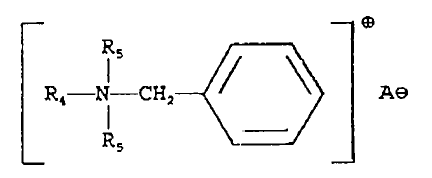 wherein each R₄ is an acyclic aliphatic C₈-C₂₂ hydrocarbon carbon group, each R₅ is a C₁-C₄ saturated alkyl or hydroxyalkyl group, and A^{⊖} is an anion;
(xvi) amide-substituted imidazolinium salts having the formula: 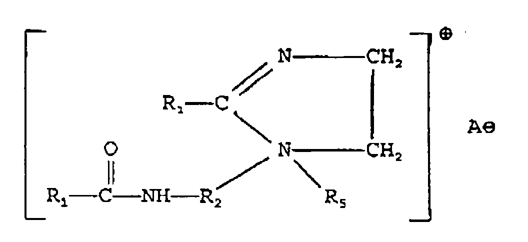 wherein each R₁ is an acyclic aliphatic C₁₂-C₂₁ hydrocarbon group, R₂ is a divalent alkylene group having 1 to 3 carbon atoms, and R₅ and A^{⊖} are as defined above or R⁵ is - H; and
(xvii) ester-substituted imidazolinium salts having the formula: 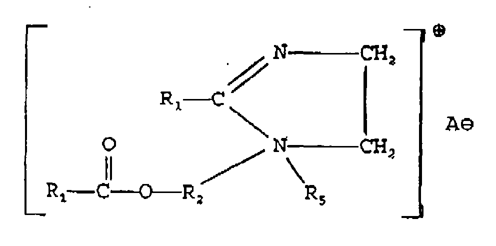 wherein R₁, R₂, R₅ and A^{⊖} are as defined above; and mixtures thereof.
   Examples of compound (xii) are the well-known dialkyldimethylammonium salts such as ditallowdimethylammonium chloride, ditallowdimethylammonium methylsulfate, di(hydrogenated tallow)dimethylammonium chloride, distearyldimethylammonium chloride, dibehenyldimethylammonium chloride. Di(hydrogenated tallow)dimethylammonium chloride and ditallowdimethylammonium chloride are preferred. Examples of commercially available dialkyldimethylammonium salts usable in the present invention are di(hydrogenated tallow)dimethylammonium chloride (trade name Adogen 442), ditallowdimethylammonium chloride (trade name Adogen 470), distearyldimethylammonium chloride (trade name Arosurf TA-100), all available from Witco Corporation. Dibehenyldimethylammonium chloride wherein R₄ is an acyclic aliphatic C₂₂ hydrocarbon group is sold under the trade name Kemamine Q-2802C by Humko Chemical Division of Witco Corporation.
   Examples of compound (xiii) are methylbis(tallowamidoethyl) (2-hydroxyethyl)ammonium methylsulfate and methylbis(hydrogenated tallowamidoethyl)(2-hydroxyethyl)ammonium methylsulfate wherein R₁ is an acyclic aliphatic C₁₅-C₁₇ hydrocarbon group, R₂ is an ethylene group, R₅ is a methyl group, R₉ is a hydroxyalkyl group and A⁻ is a methylsulfate anion; these materials are available from Witco Corporation under the trade names Varisoft 222 and Varisoft 110, respectively.
   An example of compound (xv) is dimethylstearylbenzylammonium chloride wherein R₄ is an acyclic aliphatic C₁₈ hydrocarbon group, R₅ is a methyl group and A⁻ is a chloride anion, which is sold under the trade name Varisoft SDC by Witco Corporation.
   Examples of compound (xvi) are 1-methyl-1-tallowamidoethyl-2-tallowimidazolinium methylsulfate and 1-methyl-1-(hydrogenated tallowamidoethyl)-2-(hydrogenated tallow)imidazolinium methylsulfate wherein R₁ is an acyclic aliphatic C₁₅-C₁₇ hydrocarbon group, R₂ is an ethylene group, R₅ is a methyl group and A⁻ is a chloride anion; they are sold under the trade names Varisoft 475 and Varisoft 445 respectively, by Witco.
   Additional examples of fabric softening compounds useful in the present invention include
(xviii) compounds characterized by the formula: 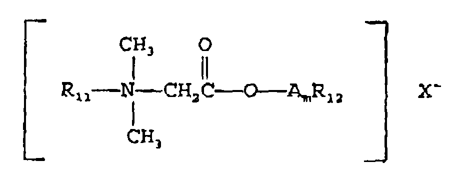 wherein
   R₁₁ is a radical selected from the group consisting of (a) straight chain aliphatic hydrocarbon radicals each of which contains from 12 through 24 carbon atoms, (b) ether radicals each of which has the structure: R₁₃O(CH₂O)_{y}-, (c) amide radicals each of which has the structure:

      R₁₄C(O)NH(CH₂)_{y}―,
   and (d) ester radicals each of which has the structure: 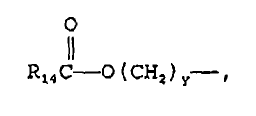
   R₁₂ is a straight chain aliphatic hydrocarbon radical containing from 12 to 32 carbon atoms,
   R₁₃ is a straight chain aliphatic hydrocarbon radical containing from 8 to 18 carbon atoms,
   R₁₄ is a straight chain aliphatic hydrocarbon radical containing from 7 to 17 carbon atoms,
   A is an alkoxy radical containing one oxygen atom and either two or three carbon atoms,
   X is an atom selected from the group consisting of bromine and chlorine,
   m is an integer of from 1 through 12, and
   y is an integer which is either 2 or 3.

   Yet additional examples of fabric softening compounds useful in the present invention include
(xix) compounds having the formula: 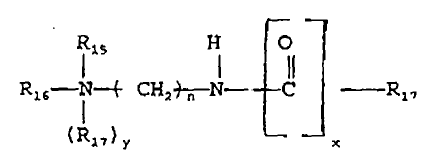 wherein each R₁₅ is selected from the group consisting of hydrogen and C₁-C₄ alkyl, each R₁₆ is selected from the group consisting of C₁-C₄ alkyl and 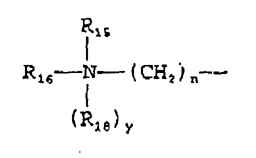 each R₁₇ is selected from the group consisting of C₈-C₂₈ alkyl and alkenyl groups, each R₁₈ is selected from the group consisting of hydrogen and C₁-C₄ alkyl, each y is 0 or 1, x is 0 or 1 and each n is from 1 to 6;
(xx) amides represented by the formula: 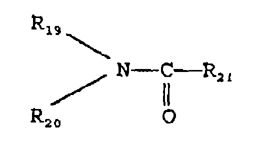 wherein R₁₉ and R₂₀ are, selected independently, C₁₋₂₂ alk(en)yl aryl, or alkyl aryl groups, R₂₁ is hydrogen, or a C₁₋₂₂ alk(en)yl, aryl or alkyl-aryl group, or is O-R₄, wherein R₂₂ is a C₁₋₂₂ alk(en)yl, aryl or alkyl-aryl group, and R₂₁ and R₂₂ possibly containing 1 to 10 ethylene oxide units, or functional groups selected from hydroxy, amine, amide, ester, and ether groups; the aryl groups being possibly derived from hetero-cyclic compounds; at least one of the R₁₉ and R₂₀ groups contains 10 or more carbon atoms; the sum of carbon atoms in R₁₉+R₂₀+R₂₁ is equal to or greater than 14. Preferably, the sum of carbon atoms in R₁₉+R₂₀ is equal to or greater than 16.
   Such species include N,N-ditallow acetamide, N,N-dicoconut acetamide, N,N-dioctadecyl propanamide, N-dodecyl, N-octadecyl acetamide, N-hexadecyl, N-dodecyl butanamide, N,N-ditallow benzamide, N,N-dicoconut benzamide, and N,N-ditallow 2-phenyl acetamide.
   Additional fabric softening compounds useful in the present invention include all ester-quaternaries, including but not limited to:
(xxi) compounds of any of the formulas 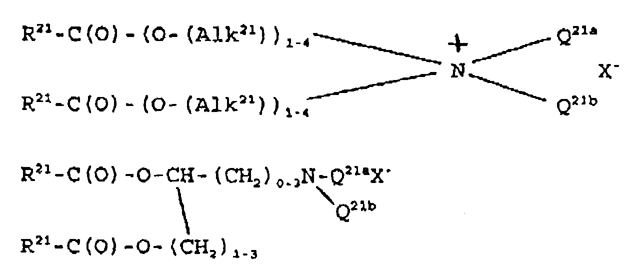 wherein
   each R²¹ is independently a saturated or unsaturated alkyl or alkylene radical containing 12 to 22 carbon atoms;
   Q^{21a} and Q^{21b} are alkyl containing 1 to 4 carbon atoms or benzyl, -CH₂CH₂OH, or -CH₂CH(OH)CH₃, or Q^{21a} can be R²¹-C(O)-(O-(Alk²¹))₁₋₄-;
   each Alk²¹ is independently C₂H₄, C₃H₆ or C₄H₈;
   R² is alkyl containing 1 to 4 carbon atoms or benzyl, -CH₂CH₂OH or -CH₂CH(OH)CH₃; and
   X⁻ is an anion;
(xxii) compounds of the formula 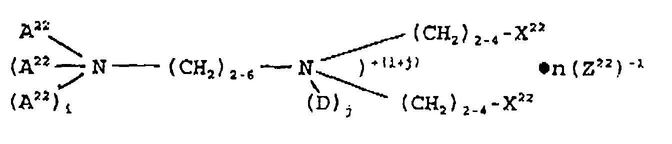 wherein each A²² is the same or different and each is alkyl containing up to 3 carbon atoms, benzyl, or H-(Alk²²-O)₁₋₃-Alk²²- wherein each Alk²² signifies -CH₂CH₂-, -CH(CH₃)CH₂-, or -CH₂CH(CH₃)-, provided further that one of the A²² can be hydrogen;
   D is methyl, ethyl, propyl, -(CH₂)₁₋₃COO⁻, benzyl or hydrogen;
   i is 0 or 1 and j is 0 or 1, provided that the sum of (i + j) is 1 or 2;
   each X²² is a straight or branched saturated or unsaturated aliphatic group containing up to 3 carbon-carbon double bonds and containing 11 to 23 carbon atoms;
   n is (two minus the number of -(CH₂)₁₋₃COO⁻ substituents present); and
   Z²² is an anion;
(xxiii) compounds of the formula

   R²³-[C(O)O(CH₂)₁₋₅]₀₋₁-C(O)NH(CH₂)₂₋₅-N(R^{23a})(R^{23b})-(CH₂)₂₋₅- OC(O)R²³X⁻

   wherein
   each R²³ is independently straight or branched alkyl or alkenyl containing 8 to 22 carbon atoms;
   R^{23a} is straight or branched alkyl or hydroxyalkyl containing 1 to 3 carbon atoms, benzyl, or -C₂H₄OC(O)R₄ wherein R⁴ is straight or branched alkyl or alkenyl containing 8 to 22 carbon atoms;
   R^{23b} is H, -CH₃, -C₂H₅ or benzyl; and
   X⁻ is an anion.
   ( 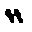 Adogen*"*, 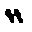 Arosurf*"* and 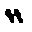 Varisoft*"* are trademarks of Witco Corp.)

In a preferred embodiment of this invention, fabric softener formulations which are clear (translucent or transparent) and easily dispersed in water can be provided by including an appropriate amount of one or more straight or branched alkyl diols containing 4 to 12 carbon atoms, and/or alkoxylates of such diols with up to 40 alkoxy units per diol moiety, wherein the alkoxylate chains are composed of alkoxy units which are ethoxy, propoxy or butoxy or mixtures thereof, and preferably ethoxy or isopropoxy.

These diols and alkoxylates correspond to the formula (T)

HO-(X-O)ₓ-R^{T}-(O-Y)_{y}-OH (T)

wherein each X and each Y is ethylene (i.e. -C₂H₄-), propylene (i.e. -C₃H₆-), or butylene (i.e. -C₄H₈-); x is 0-40; y is 0-40; the sum (x + y) is 0-40; and R^{T} is straight, branched or cyclic alkyl containing 4 to 12 carbon atoms. Preferably, R^{T} contains 7-12 or even 7-9 carbon atoms.

The alkylene residue R^{T} in formula (T) represents a saturated, straight-chain, branched-chain, or cyclic moiety containing 4 to 12 carbon atoms. It is preferred that R^{T} is branched; the term "branched" is intended to encompass structures having one side alkyl chain, more than one side alkyl chain, or one or more side alkyl chains one or more of which is itself branched. Branched structures include cyclic structures substituted with one or more alkyl groups which can be straight or branched. Examples of suitable R^{T} groups include -CH₂CH₂CH₂-, -C(CH₃)CH₂-, -CH₂CH(CH₃)CH₂-, -CH₂C(CH₃)₂CH₂-, -CH₂CH(CH₂CH₂CH₂CH₃)-, -(CH₂)₆-, 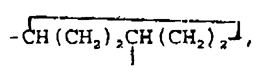 -CH₂C(CH₃)₂CH(CH(CH₃)₂)-, and -CH₂CH(CH₂CH₃)CH₂CH₂CH₂CH₂-.

In the alkoxylated diols, the number of repeating units in each poly(alkoxy) chain can be up to 40 but it is preferred that each chain contains 1 to 10 repeating alkoxy units or more preferably 1 to 5 alkoxy units. The preferred alkoxy chains are poly(ethoxy), or are composed of 1 to 2 ethoxy unite capped with a chain of 1 to 5 propoxy units.

Compounds of the formula (T) defined above are in many instances commercially available. Compounds of formula (T) can be prepared in straightforward manner familiar to those of ordinary skill in this art by obtaining or preparing the corresponding precursor diol of the formula HO-R^{T}-OH, and then alkoxylating the precursor diol with a stoichiometrically appropriate number of moles of the desired corresponding alkylene oxide, much as ethylene oxide, propylene oxide, and/or butylene oxide. In those cases where it is desired to alkoxylate only one of the hydroxyl groups on the precursor diol, in some embodiments the alkoxylation will preferentially occur at only one of the hydroxyl groups, particularly where one of them is a primary hydroxyl and the other is a secondary hydroxyl. However, in those cases where both hydroxyl groups on the precursor diol might tend to alkoxylate but alkoxylation at only one of the hydroxyl groups is desired, the hydroxyl group at which alkoxylation is desired not to occur can be protected by preliminarily reacting it with a suitable protecting group such as a lower alkyl moiety or an esterifying substituent. Thereafter, following the alkoxylation, the protecting group is removed in a known manner.

Preferred examples of compounds of the foregoing formula (T) include any one, or mixtures, of 2,2,4-trimethyl-1,3-pentane diol (referred to herein as "TMPD") and/or 2-ethylhexane-1, 3-diol, and/or the reaction product of TMPD and/or 2-ethylhexane-1, 3-diol with 1 to 10 moles of ethylene oxide, and preferably with 1 to 5 moles of ethylene oxide, as well as analogs alkoxylated with other C₃ or C₄ alkyl oxides or mixtures of any of C₂, C₃ and/or C₄ alkyl oxides. Since the diol which is alkoxylated includes one primary hydroxyl group and one secondary hydroxyl group, the alkoxylation proceeds predominantly at the primary hydroxyl group.

The compositions which contain one or more compounds of formula (1) can also contain one or a mixture of compounds of the formula (E)

R^{E1}-C(O) -R^{E2}-(OC(O)R^{E3})₀₋₁ (E)

wherein R^{E2} is straight, cyclic or branched alkyl containing 1-15 carbon atoms, and R^{E1} is optionally substituted with 1 to 3 hydroxyl groups; and wherein R^{E2} is straight, cyclic or branched alkyl containing 1 to 10 carbon atoms, and R^{E2} is substituted with 0 to 3 hydroxyl groups, and R^{E2} can optionally be substituted with a group of the structure -OC(O)-R^{E3} wherein R^{E3} is straight, cyclic or branched alkyl containing 1 to 15 carbon atoms and is optionally substituted with a hydroxyl group.

Preferred compounds of formula (E) include those wherein R^{E2} contains 2 or 3 carbon atoms, e.g. glycol and glyceryl derivative, or R^{E2} contains about 8 carbon atoms, e.g. derivatives of 2,2,4-trimethylpentane diol or of 2-ethyl hexane diol. Preferred compounds of formula (E) include 2,2,4-trimethyl-1,3-pentane diol monoisobutyrate, hydroxypivalyl hydroxypivalate, and the monoester of TMPD with hydroxypivalic acid.

Formulations can also contain what may be termed aesthetic additives present in small amounts, generally up to 1 wt.%, to provide properties such as fragrance, preservative, viscosity control, and/or color.

The formulations according to the present invention exhibit highly satisfactory viscositites, generally as pourable and even sprayable fluids.

The following examples are provided for purposes of further description of the present invention. The examples are not intended to limit the scope of that which is regarded as the invention.

### EXAMPLE 1

1,6-Hexamethylene diamine was reacted with 4 moles of ethylene oxide per mole of diamine. The ethoxylate was esterified in two different runs with a mixture of C₁₄-C₁₈ fatty acids (2 moles of fatty acid per mole of ethoxylate) following which the diamine was quaternized with 2 moles of dimethyl sulfate per mole of diamine. In Run (1-A) the fatty acids were a commercial mixture of tallow fatty acids and in Run (1-B) the fatty acids were obtained from canola oil.

### EXAMPLE 2

This example illustrates formulations of compounds according to formula (1) in conventional solvent systems. A product was made by thoroughly blending the quaternary and solvent, then blending in the fragrance and then the water. The components and the amounts thereof were:

| Component | Amount (wt.%) |
|---|---|
| Product of Run 1-B (85 wt.% in isopropanol) | 23.5 |
| Solvent (hexylene glycol) | 18-20 |
| Fragrance | 1.0 |
| Deionized water | 55.5 |
| Dye/Preservative | q.s. |

The resulting formulations had a cloud point of 45°C., a melting point above 10°C., and a Gardner color of above 0.5. The quaternary product of Run 1-B exhibits little or no gelation upon addition to water.

The above formulation was repeated except using a 50:50 (wt.) mixture of products prepared in accordance with Runs 1-A and 1-B. The resulting formulation has a cloud point of 50-55°C. and exhibits satisfactory properties.

### EXAMPLE 3

This example illustrates that clear formulations can be made which contain a conventional quaternary ammonium fabric softener compound together with one or a mixture of compounds of formula (1) as defined herein. This discovery is particularly unexpected in that many conventional quaternary ammonium fabric softener actives, such as the di(C₈-C₂₂ alkyl/alkenyl) di(C₁-C₄ alkyl) quaternary ammonium compounds, like the one employed in this example, cannot readily be formulated into a clear composition in an acceptable amount, using existing solvent systems or solvent/coupling agent systems.

A composition was made using the procedure used in Example 2. The components and the amounts thereof were:

| Component | Amount (wt.%) |
|---|---|
| Product of Run 1-B, 85 wt.% in isopropanol | 14.1 |
| Ditallow dimethyl ammonium chloride ( 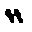 Adogen 470", Witco Corp.), 75 wt.% in isopropanol | 10.7 |
| Hexylene glycol | 24.0 |
| Deionized water | 61.9 |
| Fragrance | 1.0 |
| Dye/preservative | q.s. |

The resulting product has a cloud point of 45-50°C, and a melting point of 27°C.

Its color is less than 0.5 Gardner.

Additional formulations employing another solvent system include the following, made by the procedure disclosed in Example 2.

### EXAMPLE 4

| Component | Amount (wt.%) |
|---|---|
| Product of Run 1-B | 23.5 |
| TMPD | 7.5 |
| Hexylene Glycol | 7.5 |
| Fragrance | 0.5-1 |
| Preservative | q.s. |
| Deionized water | 60.5 |

### EXAMPLE 5

| Component | Amount (wt.%) |
|---|---|
| Product of Run 1-A | 11.8 |
| Product of Run 1-B | 11.8 |
| TMPD | 7.5 |
| Hexylene Glycol | 7.5 |
| Fragrance | 0.5-1. |
| Preservative | q.s. |
| Deionized water | 60.5 |

More generally, exemplary formulation guidelines for fabric softener compositions which include polyester polyquaternary products conforming to formula (1), include the following:

### Clear Polyester Polyquaternary in Conventional solvent(s)

| Component | Amount (wt%) |
|---|---|
| Product(s) conforming to formula (1) | 15-20 |
| Solvent(s) (e.g. isopropanol, hexylene glycol | 20-30 |
| Dye, preservative | As needed |
| Deionized Water | To 100. |

### Clear Polyester Polyquaternary with Coupling Agent

| | |
|---|---|
| Product(s) conforming to formula (1) | 15-25 |
| Coupling agent(s) (e.g. diol or alkoxylate of formula (T) and/or hydroxyester of formula (E)) | 10-15 |
| Acid (e.g. HCl) | to pH 2.5-4. |
| Dye, preservative | As needed |
| Deionized water | To 100. |

## Claims

1. Compounds of the formula (1) 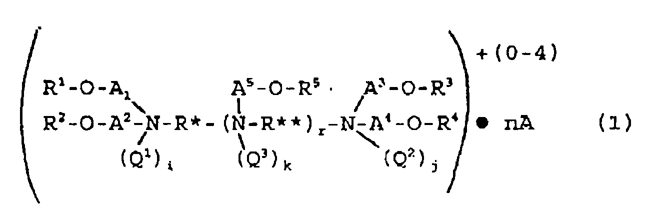 wherein each of R* and R** is independently a linear, branched or cyclic alkyl group containing 2 to 12 carbon atoms, wherein no two nitrogen atoms are separated by fewer than 2 carbon atoms;
each of A¹, A², A³, A⁴ and each occurrence of A⁵ is independently straight or branched alkyl containing 2 to 4 carbon atoms;
each of R¹, R², R³, and R⁴ and each occurrence of R⁵ is independently -H or R^{A}C(=O)- wherein R^{A} is straight or branched alkyl or alkenyl containing 9 to 21 carbon atoms and 0 to 4 carbon-carbon double bonds; provided that at least one of R¹, R², R³, R⁴ or R⁵ is R^{A}C(=O)-;
each of Q¹, Q² and Q³ is -H,-CH₃, -C₂H₅, or - CH₂CH₂COOH;
r is 0-2;
i is 0-1, j is 0-1, each k is 0-1 and the sum of (i+j+k) is 0-4; or a 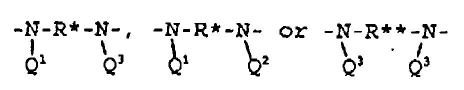 moiety is 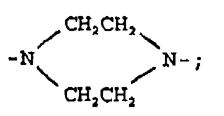
A is a monovalent anion; and n is the number of moles of A needed to give the compound of formula (A) zero net charge.

2. A compound of the formula (Q) 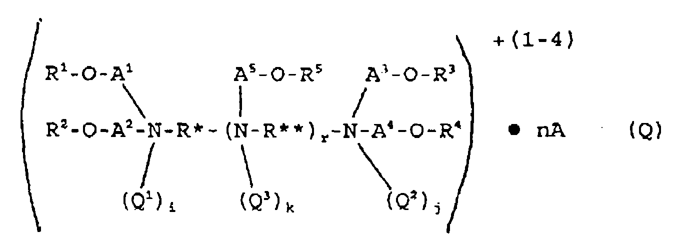 wherein each of R* and R** is independently a linear, branched or cyclic alkyl group containing 2 to 12 carbon atoms, wherein no two nitrogen atoms are separated by fewer than 2 carbon atoms;
each of A¹, A², A³, A⁴ and each occurrence of A⁵ is independently straight or branched alkyl containing 2 to 4 carbon atoms;
each of R¹, R², R³, and R⁴ and each occurrence of R⁵ is independently -H or R^{A}C(=O)- wherein R^{A} is straight or branched alkyl or alkenyl containing 9 to 21 carbon atoms and 0 to 4 carbon-carbon double bonds; provided that at least one of R¹, R², R³, R⁴ or R⁵ is R^{A}C(=O)-;
each of Q¹, Q² and Q³ is -CH₃, -C₂H₅, or - CH₂CH₂COOH;
r is 0-2;
i is 0-1, j is 0-1, each k is 0-1 and the sum of (i+j+k) is 1-4; or a 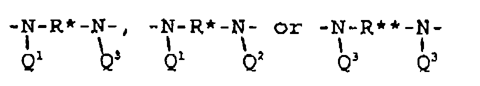 moiety is 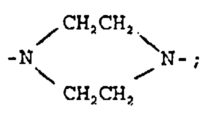
A is a monovalent anion; and n is the number of moles of A needed to give the compound of formula (Q) zero net charge.

3. A compound according to claim 1 wherein R^{*} is -(CH₂)₂₋₆-.

4. A compound according to claim 1 wherein R^{*} is -(CH₂)₂-.

5. A compound according to claim 1 wherein R^{*} is -(CH₂)₆-.

6. A compound according to claim 1 which is substituted with one R^{A}C(O)- group.

7. A compound according to claim 1 which is substituted with two R^{A}C(O)- groups.

8. A compound according to claim 1 which is substituted with three R^{A}C(O)- groups.

9. A compound according to claim 1 which is substituted with four R^{A}C(O)- groups.

10. A compound according to claim 1 wherein r is 0.

11. A compound according to claim 1 wherein r is 1.

12. A compound according to claim 1 wherein r is 2.

13. A mixture of two or more compounds of formula (1) as defined in claim 1, wherein the compounds and the amounts thereof are selected such that on average per mole of compounds of formula (1) in said mixture, the number of substituents of the formula R^{A}C(=O)- is 1-4.

14. A mixture of two or more compounds of formula (1) as defined in claim 1, wherein the compounds and the amounts thereof are selected such that on average per mole of compounds of formula (1) in said mixture, the number of substituents of the formula R^{A}C(=O)- is 2-3.

15. A mixture of two or more compounds of formula (1) as defined in claim 1, wherein the compounds and the amounts thereof are selected such that on average per mole of compounds of formula (1) in said mixture, the sum of (i+j+k) is 1.5-3.0.

16. A mixture of two or more compounds of formula (1) as defined in claim 1, wherein R is zero, and wherein the compounds and the amounts thereof are selected such that on average per mole of compounds of formula (1) in said mixture, the sum of (i+j) is 1.5-2.0.

17. A mixture according to claim 13 wherein R^{*} is -(CH₂)₂₋₆-.

18. A mixture according to claim 13 wherein R^{*} is -(CH₂)₂-.

19. A mixture according to claim 13 wherein R^{*} is -(CH₂)₆-.

20. A liquid composition useful as a fabric softener and comprising
(a) a polyquaternary component selected from the group consisting of compounds of the formula (1) 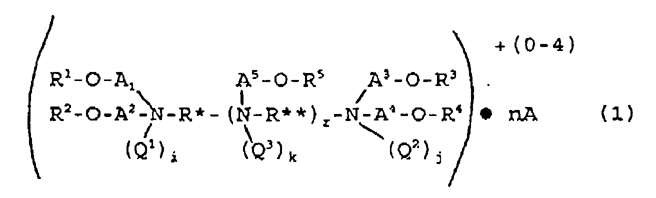 wherein each of R* and R** is independently a linear, branched or cyclic alkyl group containing 2 to 12 carbon atoms, wherein no two nitrogen atoms are separated by fewer than 2 carbon atoms;
each of A¹, A², A³, A⁴ and each occurrence of A⁵ is independently straight or branched containing 2 to 4 carbon atoms;
each of R¹, R², R³, and R⁴ and each occurrence of R⁵ is independently -H or R^{A}C(=O)- wherein R^{A} is straight or branched alkyl or alkenyl containing 9 to 21 carbon atoms and 0 to 4 carbon-carbon double bonds; provided that at least one of R¹, R², R³, R⁴ or R⁵ is R^{A}C(=O)-;
each of Q¹, Q² and Q³ is -H, -CH₃, -C₂H₅, or - CH₂CH₂COOH;
r is 0-2;
i is 0-1, j is 0-1, each k is 0-1 and the sum of (i+j+k) is 0-4; or a 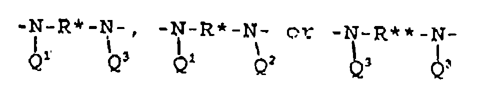 moiety is 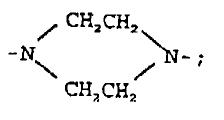
A is a monovalent anion; and n is the number of moles of A needed to give the compound of formula (1) zero net charge;
(b) water; and
(c) optionally one or more solvents, cosolvents, or both.
